# EUROPEAN PATENT SPECIFICATION

(11) **EP 4 218 852 B1**
(45) Date of publication and mention of the grant of the patent: **18.03.2026**
(21) Application number: 22305103.8
(22) Date of filing: 28.01.2022
(51) Int. Cl.: A61M 5/00

(54) **DEVICE AND SYSTEM FOR TRANSPORTING MEDICAL DEVICES**
VORRICHTUNG UND SYSTEM ZUM TRANSPORTIEREN VON MEDIZINISCHEN VORRICHTUNGEN
DISPOSITIF ET SYSTÈME DE TRANSPORT DE DISPOSITIFS MÉDICAUX

(43) Date of publication of application: 02.08.2023
(73) Proprietor: Becton Dickinson France, 38800 Le Pont de Claix (FR)
(72) Inventor: POUGET, Gaelle, 38100 GRENOBLE (FR); CHASSIN DE KERGOMMEAUX, Liliane, 38000 GRENOBLE (FR); VAZ, Victor, 38760 VARCES-ALLIERES-ET-RISSET (FR)
(74) Representative: Regimbeau

(56) References cited:
- US-A1- 2014 308 181
- US-A1- 2017 183 113
- US-A1- 2021 170 092

## Description

### TECHNICAL FIELD

The present disclosure relates generally to a device, which is commonly referred to in the art as a nest, for transporting medical containers, such as syringes, and to a system including such device.

### BACKGROUND

Medical containers, such as pre-fillable or prefilled syringes, may be transported from one site to another site. For instance, medical containers may be transported from a manufacturing site to a site at which the medical containers may be filled with a medicinal fluid. By way of further example, the medical containers may be manufactured and filled at the same site and subsequently transported to a storage site in a sterile packaging. During transportation, the medical containers may be supported by a holding device, which may be referred to as a nest. The holding device may be disposed within a packaging having an opening allowing for placement of the holding device therein and subsequently sealed by a sealing cover. The container may be referred to as a tub. Documents US 20170183113, US 2021170092 and US 20140308181 describe such transportation devices.

External dimensions of the holding devices being slightly smaller than inner dimension of the packaging for ease of the assembly, the holding device may be susceptible to moving relative to the packaging during transportation. As a result of such movement during long-distance transport, the holding device rubs against an inner surface of the packaging and may wear away material of the packaging or of the holding device so as to create particles of the packaging material and/or the holding device material. Because the packaging is sealed by the sealing cover, the particles remain inside the packaging throughout the remainder of transportation and storage. Such particles may be dispersed within the packaging and thus disposed within or on the medical containers stored inside the packaging. When the medical containers arrive at the destination, the medical containers are intended to be in a ready-to-use condition. By way of non-limiting example, when the medical containers are pre-fillable syringes, the syringes are intended to be in a clean and sterile condition upon arrival at the filling site so that the syringes may be filled with medicinal fluid. If the particles of the packaging material and/or of the holding device material are dispersed within or on the syringes, the syringes are not in a ready-to-use condition and must be cleaned prior to filling in order to avoid contamination of the medicinal fluid with which the medical containers will be filled. Accordingly, there is a need for a packaging solution that would inhibit or altogether prevent the creation of particles within the packaging.

### BRIEF SUMMARY

An objective of the present disclosure is to provide a device for supporting a plurality of medical containers solving the above-mentioned drawbacks. The device comprises a plate having an upper surface, a lower surface, and a sidewall defining a perimeter of the plate and extending between the upper surface and the lower surface. The sidewall comprises four edges connected by corners. A longitudinal axis of the plate extends perpendicular to the upper surface. The plate defines a plurality of openings extending therethrough from the upper surface to the lower surface. Each opening is configured to retain a medical container therein. The plate is dimensioned to be disposed in a packaging for transporting a plurality of medical containers such that a distance measured between opposing edges of the sidewall is less than a distance measured between opposing sidewalls of an interior surface of the packaging. The device further comprises at least two positioning elements coupled at a first end thereof to the plate. Each of the positioning elements have a second end opposite the first end detached from the plate.

When the plate is disposed in the packaging, each positioning element forms an acute angle relative to the longitudinal axis of the plate and extends a lateral distance beyond the sidewall of the plate such that each of the positioning elements contacts the interior surface of the sidewall of the packaging when the plate is disposed therein.

Typically, the first of the at least two positioning elements is at least partly disposed on first edge of the four edges, the second of the at least two positioning elements is at least partly disposed on a second edge of the four edges, the first edge and second edge being adjacent to each other.

According to the invention, the first of the at least two positioning elements is exclusively disposed on a first edge of the four edges, the second of the at least two positioning elements is exclusively disposed on a second edge of the four edges, and the first edge and second edge are adjacent to each other.

In another not claimed embodiment, the first of the at least two positioning elements is disposed at a first corner, the second of the at least two positioning elements is disposed at a second corner, and the first and second corners are diagonally opposite each other. In this embodiment, when a positioning element is disposed at a corner, it is disposed at the intersection of two adjacent edges, and thus it disposed partly on a first edge and partly on a second edge adjacent to the first edge.

Certain preferred but non-limiting features of the device described above are the following, taken individually or in combination:
the positioning element is a rigid element configured to be displaced such that the acute angle of the positioning element is variable by 1° when the plate having the positioning elements thereon is disposed within the packaging and when the plate is exterior to the packaging;
the positioning element is a flexible element configured to be displaced such that the acute angle of the positioning element is variable by 65° to 80° when the plate having the positioning elements thereon is disposed within the packaging and when the plate is exterior to the packaging;
the positioning elements are formed integrally with the plate;
at least one positioning element is coupled to each edge of the sidewall; and/or
the device comprises four positioning elements, and one positioning element is coupled to each corner of the sidewall.

Another objective of the present disclosure is to provide a packaging system for receiving a plurality of medical containers comprising a packaging comprising a sidewall extending from a bottom wall, an interior surface of the sidewall being stepped so as to define a ledge. The packaging system also comprises the device comprising the plate having the at least two positioning elements as previously described disposed in the packaging. The lower surface of the plate is disposed on the ledge and the positioning elements are disposed on the interior surface of the sidewall.

Certain preferred but non-limiting features of the packaging system described above are the following, taken individually or in combination:
a surface of the positioning element disposed on the interior surface of the sidewall has a curvature at corners that corresponds to a curvature of the interior surface of the plurality of sidewalls;
a minority of a surface area of the surface of the positioning element is disposed against the interior surface of the sidewall;
an axial dimension of each positioning element is less than or equal to a longitudinal dimension measured between the ledge and an uppermost surface of the packaging;
the plate comprising the positioning elements engages the interior surface of the sidewalls of the packaging by interference fit; and/or
the device further comprises a plurality of chimneys, each chimney extends circumferentially about an opening of the plurality of openings and extends axially over the upper surface of the plate, each chimney configured to retain a medical container therein.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIGS. 1A-1C are top, schematic views of a device for supporting a plurality of medical containers;
FIGS. 2-4 are partial, cross-sectional views of the device of FIGS. 1A-1C relative to a packaging;
FIGS. 5A-5B are top, schematic views of another device for supporting a plurality of medical containers;
FIG. 6 is a partial, perspective view of the device of FIGS. 5A-5B;
FIG. 7 is a partial, cross-sectional view of the device of FIGS. 5A-5B relative to a packaging; and
FIG. 8 is a perspective view of the device of FIGS. 5A-5B disposed in a packaging.

### DETAILED DESCRIPTION

As used herein, relational terms, such as "first," "second," "top," "bottom," "upper," "lower," "over," "under," "on," "interior," "exterior," and the like, are used for clarity and convenience in understanding the disclosure and accompanying drawings and do not connote or depend on any specific preference, orientation, or order, except where the context clearly indicates otherwise

As used herein, the terms "axial," "axially," "longitudinal," and "longitudinally" generally mean and refer to a direction along or parallel to a longitudinal axis of an element(s) of the plate and packaging described herein.

As used herein, the terms "lateral" and "laterally" generally mean and refer to a direction perpendicular to the central, longitudinal axis of the element(s) of the plate and packaging described herein.

As used herein, the term "about" in reference to a given parameter is inclusive of the stated value and has the meaning dictated by the context (e.g., it includes the degree of error associated with measurement of the given parameter).

As used herein, the term "and/or" includes any and all combinations of one or more of the associated listed items.

As used herein, the term "configured" refers to a size, shape, material composition, material distribution, orientation, and arrangement of one or more of at least one structure and at least one apparatus facilitating operation of one or more of the structure and the apparatus in a pre-determined way.

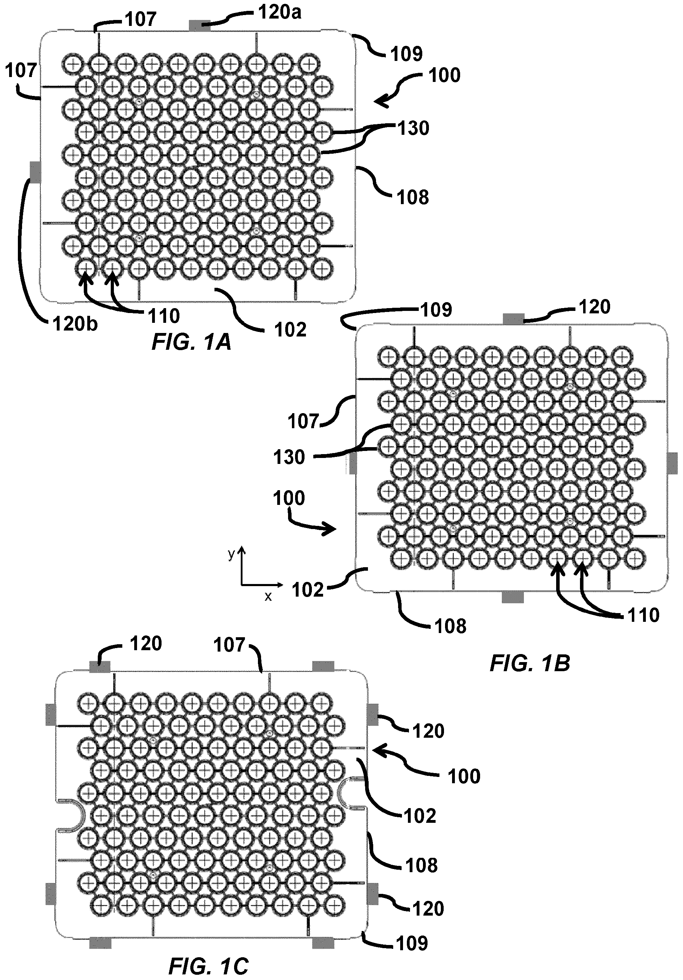

FIGS. 1A-1C illustrate a device 100 configured to support a plurality of medical containers therein. The device 100 may be referred to in the art generally as a nest. The medical containers may be vials, syringes, such as pre-fillable or prefilled syringes, or any medical element which may be transported in a nest, such as stoppers or syringe gaskets. The medical containers are configured, after filling, to contain a medical fluid therein.

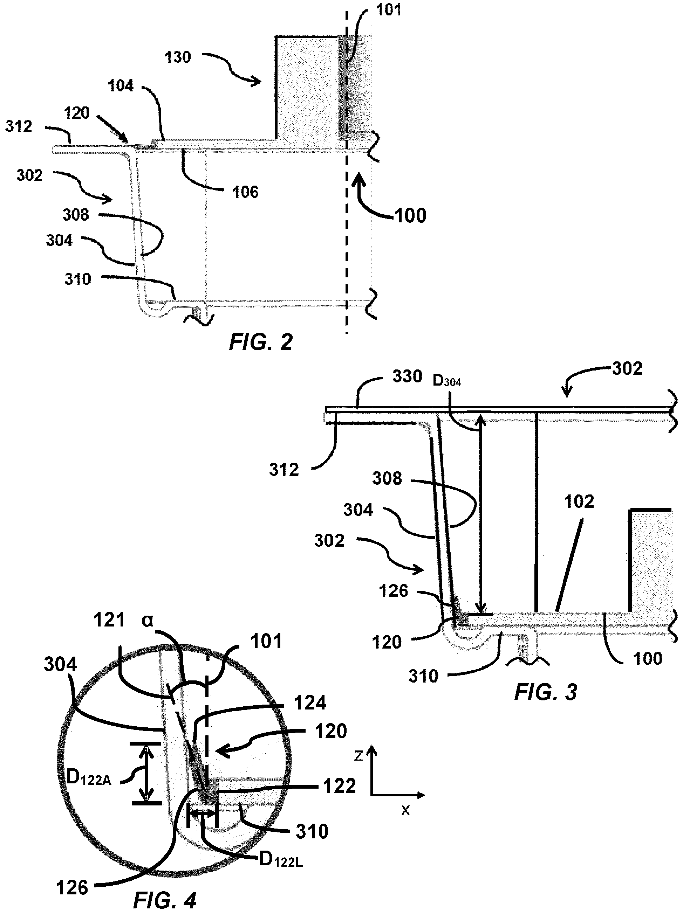

The device 100 may comprise a plate 102 having an upper surface 104, a lower surface 106 (seen in FIG. 2), and a sidewall 108 extending between the upper surface 104 and the lower surface 106.

The sidewall 108 defines a perimeter of the plate 102. The sidewall 108 comprises four edges 107 connected by corners 109. The corners 109 may be rounded or pointed. The edges 107 may comprise planar surfaces. Such surfaces of opposing edges 107 may extend parallel to each other.

A longitudinal axis 101 of the plate 102 extends perpendicular to (e.g., normal to) the upper surface 104.

The plate 102 defines a plurality of openings 110 extending therethrough from the upper surface 104 to the lower surface 106. Each opening 110 is configured for a medical container to pass therethrough.

The plate 102 may also comprise a plurality of chimneys 130 that protrude axially from the upper surface 104. Each chimney 130 extends at least partially circumferentially about a corresponding opening 110. Each chimney 130 may be a generally cylindrical structure. Each chimney 130 defines an opening within which a medical container may be retained. Each chimney 130 may have a centrally extending longitudinal axis that is parallel with the longitudinal axis 101 of the plate 102. Each chimney 130 may be integrally formed with the plate 102.

The device 100 comprises at least two positioning elements 120. Each positioning element 120 extends between a first end 122 and a second end 124 opposite the first end 122. The first end 122 is coupled to the plate 102. The second end 124 is detached from the plate 102.

A first positioning element 120a is at least partly located (e.g., disposed) on a first edge 107. A second positioning element 120b is at least partly located on a second edge 107.

Typically, in a first embodiment, the first positioning element 120a may be exclusively (e.g., fully) located on a first edge 107. The second position element 120b may be exclusively located on a second edge 107 that is adjacent to the first edge 107. FIG. 1A illustrates an embodiment including two positioning elements 120 in such a configuration.

In further embodiments, the device 100 may include four positioning elements 120, as illustrated in FIG. 1B. One positioning element 120 may be positioned on each edge 107. In some embodiments, the positioning elements 120 may be distributed such that the positioning elements 120 on opposing edges 107 are disposed directly opposite each other. In other words, when the device 100 comprises four positioning elements 120, the position elements may be respectively located around the middle of each of the four edges 107 of the device 100 (e.g., intermediately between corners 109).

In yet further embodiments, the device 100 may include eight positioning elements 120, as illustrated in FIG. 1C. Two positioning elements 120 may be positioned on each edge 107. In some embodiments, the positioning elements 120 may be distributed such that one positioning element 120 on a given edge 107 is located directly opposite another positioning element 120 on a directly opposite edge 107.

In each of the foregoing embodiments, the positioning elements 120 may be positioned exclusively only edges 107 of the plate 102 such that the positioning elements 120 do not extend along the corners 109.

The location of the positioning elements 120 on at least two adjacent edges 107 as explained with references to FIGS. 1A-1C limits movement of the device 100 in two directions perpendicular to the longitudinal axis 101, including the x-direction and the y-direction as indicated by the coordinate system of FIG. 1B, when the device 100 is disposed within a packaging, or tub, and submitted to long-distance transport.

The device 100 is dimensioned to be disposed in a packaging configured to transport a plurality of medical containers. FIG. 2 illustrates partial view of a packaging 302 in which the device 100 may be disposed. The device 100 and the packaging 302 collectively form a packaging assembly for transporting a plurality of medical containers.

The packaging 302 comprises a sidewall 304 defining a perimeter thereof and extending from a bottom wall 306. An interior of the packaging 302 is hollow and is defined by an interior surface of the bottom wall 306 and an interior surface 308 of the sidewall 304.

The sidewall 304 may be stepped so as to define a ledge 310. The ledge 310 provides a horizontal surface configured to support the device 100. When the device 100 is disposed in the packaging 302, the lower surface 106 of the device 100 is disposed on the ledge 310. The ledge 310 may extend continuously or discontinuously along a perimeter of the interior surface 308.

A flange may be provided at an end of the sidewall 304 opposite to which the bottom wall 306 is located. The flange defines an uppermost surface 312 of the sidewall 304. The flange may extend continuously about the perimeter of the sidewall 304.

The flange further defines a perimeter of an opening 314 through which the device 100 may be disposed within the packaging 302.

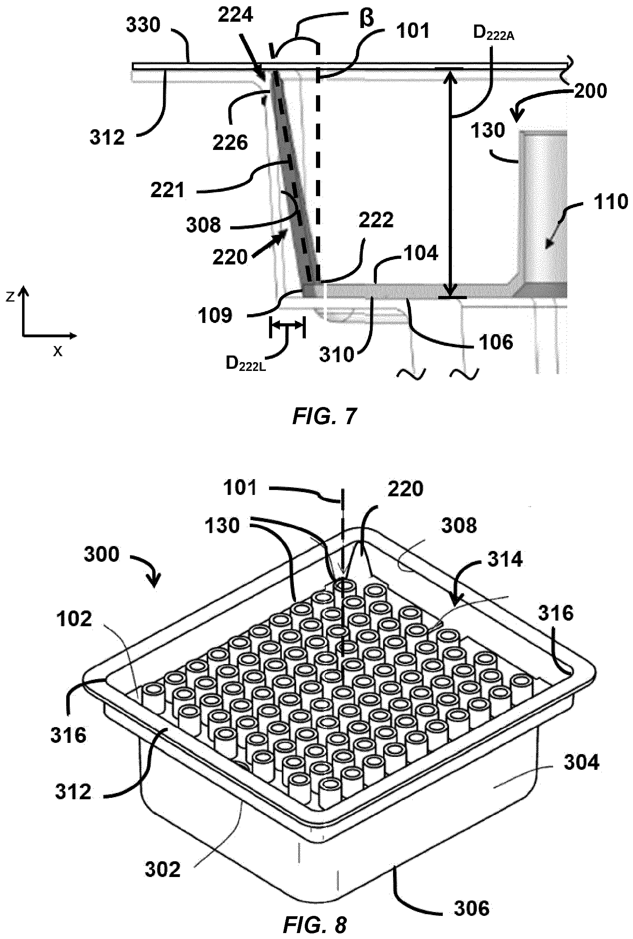

At least a portion of the inner surface 308 of the sidewalls 304 may be sloped. More particularly, the inner surface 308 of the sidewall 304 extending between the ledge 310 and the uppermost surface 312 may be sloped. The slope of the sidewall 304 is best illustrated in FIGS. 2 and 7.

As previously stated, the device 100 is dimensioned to be disposed in the packaging 302 through the opening 314. More particularly, the plate 102 is dimensioned such that a distance measured between opposing edges 107 of the sidewall 108 (i.e., a lateral dimension being either a width or length of the plate 102 excluding the positioning element 120) is less than a distance measured between corresponding opposing interior surfaces 308 of the sidewall 304.

The positioning element 120 has an axis 121 extending between the first end 122 and the second end 124. The axis 121 of the positioning element 120 forms an angle α relative to the longitudinal axis 101 of the plate 102.

The positioning element 120 may be a flexible element. As used herein, the term "flexible element" refers to a positioning element 120 for which the angle α is movable by up to 90° relative to a first position when the device 100 is exterior to the packaging 302 and a second position when the device 200 is interior to the packaging 302. More particularly, the positioning element 120 is movable from the first position when the device 100 is exterior to the packaging 302 to a second position when the device is interior to the packaging 302 by 65° to 80°, inclusive.

Prior to disposing the device 100 within the packaging 302, the angle α of the positioning element 120 extends at 90° relative to the longitudinal axis 101, as illustrated in FIG. 2. As the device 100 is inserted through the opening 314 toward the ledge 310, the angle α decreases as an exterior surface 126 of the positioning element 120 engages with the interior surface 308 of the sidewall 304.

When the device 100 is disposed within the packaging 302 such that the lower surface 106 of the plate 102 is disposed on the horizontal surface of the ledge 310, as illustrated in FIGS. 3 and 4, the angle α of the positioning element 120 forms an acute angle relative to the longitudinal axis 101. The acute angle α may extending a range from 10° to 25°, inclusive, and, more preferably, in a range from 15° to 20°, inclusive.

The flexibility of the positioning elements 120 allows for the lateral dimensions of the device 100 to be reduced as the device 100 is disposed in the packaging 302. The flexibility of the positioning elements 120 also allows the device 100 to be removed from the packaging 302.

A given positioning element 120 projects axially (i.e., parallel to the longitudinal axis 101) beyond the upper surface 104 and laterally (i.e., perpendicular to the longitudinal axis 101) beyond the sidewall 108. Accordingly, each positioning element 120 has an axial dimension D_{122A}, which is measured as the positioning element 120 projects from the lower surface 106 to the second end 124 of the positioning element 120 and a lateral dimension D_{122L}, which is measured as the positioning element 120 projects from an edge 107 of the sidewall 108 at which the first end 122 of the positioning element 120 is coupled to the second end 124 of the positioning element 120.

Prior to disposing the device 100 within the packaging 302, the lateral dimension D_{122L} of at least one positioning element 120 and the lateral dimension of the plate 102, when combined, are greater than the lateral dimension (i.e., distance measured between two opposing interior surfaces 308 of the sidewall 304 excluding the positioning elements 120) of the packaging 302.

When the device 100 is disposed in the packaging 302 and on the ledge 310, the device 100 and the packaging 302 are engaged by interference fit.

In some embodiments, such as the embodiment of FIG. 1A, when the device 100 is disposed within the packaging 302, a space may be located between the interior surface 308 of the sidewall 304 and at least two edges 107 and at least one corner 109 of the sidewall 108 of the plate 102. In other embodiments, such as the embodiments of FIGS. 1B and 1C, when the device 100 is disposed within the packaging 302, a space may be located between the interior surface 308 of the sidewall 304 and each edge 107 and corner 109 of the sidewall 108 of the plate 102. In such embodiments, there is no contact between the sidewall 304 and the sidewall 108.

When the device 100 is disposed within the packaging 302, a minority of a surface area of the exterior surface 126 of the positioning element 120 contacts the interior surface 308 of the sidewall 304.

The exterior surface 126 may be planar as the positioning element 120 extends along the edge 107. The planar exterior surface 126 corresponds to the planar surface of the sidewall 304 of the packaging 302 that the positioning element 120 contacts.

The engagement of the positioning elements 120 and, optionally, the sidewall 108 of plate 102 for embodiments lacking a positioning element 120 on each edge 107 with the inner surface 308 of the packaging 302 prevents the device 100 from moving in a direction perpendicular to the longitudinal axis 101 of the plate 102. As lateral movement between the device 100 and the packaging 302 is avoided, the device 100 and the packaging 302 do not rub inagainst each other so as to create particles of the packaging material and/or the device material.

The interference fit between the device 100 and the packaging 302 also prevents or limits movement of the device 100 along the Z direction through friction with the packaging 302. Preferably, both lateral and longitudinal movements between the device 100 and the packaging 302 are avoided.

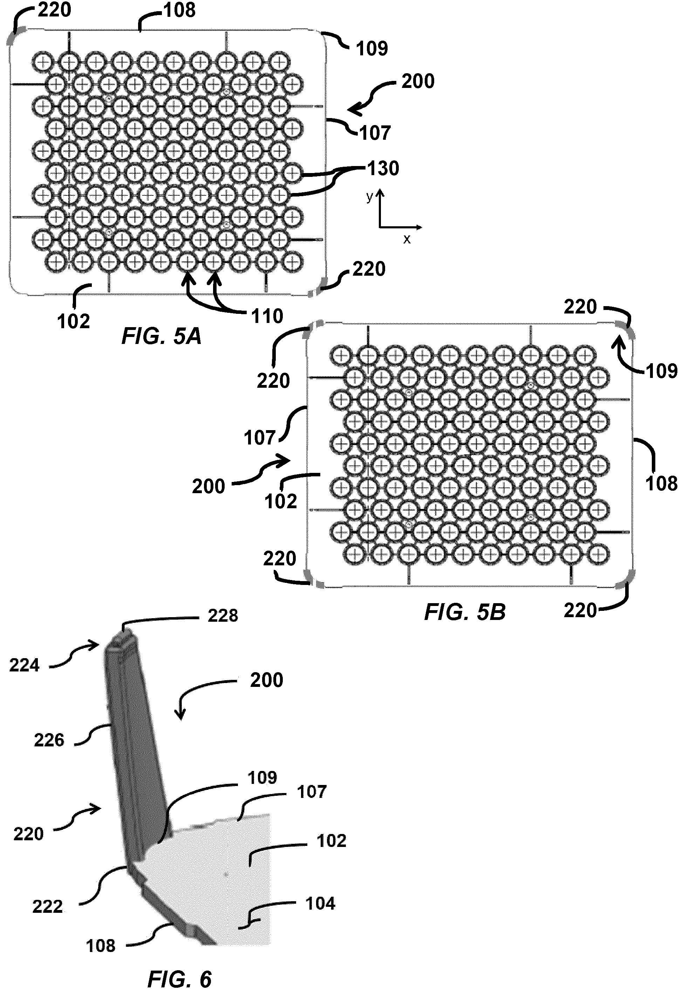

FIGS. 5A and 5B illustrate a device 200 configured to support a plurality of medical containers therein.

The device 200 comprises the plate 102 having the upper surface 104, the lower surface 106, and the sidewall 108 extending between the upper surface 104 and the lower surface 106, as previously described herein.

The device 200 comprises at least two positioning elements 220. As illustrated in FIG. 6, each positioning element 220 may extend between a first end 222 and a second end 224 opposite the first end 222. The first end 222 is coupled to the plate 102. The second end 224 is detached from the plate 102.

A first positioning element 220a may be located at a first corner 109. A second position element 220b may be located at a second corner 109 that is diagonally opposite to the first corner 109. FIG. 5A illustrates an embodiment including two positioning elements 220. When a positioning element 220 is positioned at a corner 109 of the device 200, the positioning element is partly disposed on two adjacent edges 107.

In further embodiments, the device 200 may include four positioning elements 220, as illustrated in FIG. 5B. One positioning element 220 may be positioned at each corner 109.

The location of the at least two positioning elements 220 on diagonally opposed corners 109 limits movement of the device 200 in directions perpendicular to the longitudinal axis 101, including the x-direction and the y-direction as indicated by the coordinate system of FIG. 5A, when the device 200 is disposed within the packaging 302.

Each positioning element 220 has an axis 221 extending between the first end 222 and the second end 224. The axis 221 of the positioning element 220 forms an angle β relative to the longitudinal axis 101 of the plate 102.

The positioning element 220 may be a rigid element. As used herein, the term "rigid element" refers to a positioning element 220 for which the angle β is movable by up to 5° and, more particularly, by 1° between a first position when the device 200 is exterior to the packaging 302 and a second position when the device 200 is interior to the packaging 302.

Prior to disposing the device 200 within the packaging 302, the angle β of the positioning element 220 is about 5° greater than an angle of the sloped sidewall 304 of the interior surface 308 of the packaging 302 (e.g., an angle measured between a line tangent to the interior surface 308 of the sidewall 304 and the longitudinal axis 101). In some embodiments, the angle β of the positioning element 220 extends in a range from 31° to 46°, inclusive, and, more preferably, in a range from 36° to 41°, inclusive, relative to the longitudinal axis 101. As the device 200 is inserted through the opening 314 and toward the ledge 110, the angle β decreases as an exterior surface 226 of the positioning element 220 engages with the interior surface 308 of the sidewall 304.

When the device 200 is disposed within the packaging 302 such that the lower surface 106 of the plate 102 is disposed on the horizontal surface of the ledge 310, the angle β of the positioning element 220 forms an acute angle relative to the longitudinal axis 101. This acute angle β is about 4° larger than the angle between the interior surface 308 of the sidewall 304 and the longitudinal axis 101. Accordingly, the acute angle β may extend a range from 30° to 45°, inclusive, and, more preferably, in a range from 35° to 40°, inclusive, relative to the longitudinal axis 101, as illustrated in FIG. 7.

The flexibility of the positioning elements 220 allows the lateral dimensions of the device 100 to be reduced as the device 200 is disposed in the packaging 302. The flexibility of the positioning elements 220 also allows the device 200 to be removed from the packaging 302.

As discussed with reference to the device 100, the plate 102 is dimensioned such that a distance measured between opposing edges 107 of the sidewall 108 (i.e., a lateral dimension being either a width or length of the plate 102 excluding the positioning elements 220) is less than a distance measured between corresponding opposing interior surfaces 308 of the sidewall 304. The plate 102 is also dimensioned such that a distance measured between opposing corners 109 of the sidewall 108 (i.e., a lateral dimension being a diagonal of the plate 102) is less than a distance measured between diagonally opposing corners 316 of the packaging 302.

A given positioning element 220 projects axially beyond the upper surface 104. The positioning element 220 projects laterally beyond the sidewall 108 and, more particularly, laterally beyond the corner 109. The positioning element 220 has an axial dimension D_{222A}, which is measured as the position element 220 projects between the lower surface 106 and the second end 224 of the positioning element 220 and a lateral dimension D_{222L}, which is measured as the positioning elements 220 projects between a corner 109 of the sidewall 108 to which the first end 222 of the positioning element 220 is coupled and the second end 224 of the positioning element 220.

The axial dimension D_{222A} is less than or equal to an axial dimension D₃₀₄ measured between the horizontal surface of the ledge 310 and the uppermost surface 312 of the sidewall 304. The longitudinal dimension D₃₀₄ is measured in a direction normal to the interior surface of the bottom wall 306.

When the axial dimension D_{222A} is equal to the axial dimension D₃₀₄, the second end 224 of the positioning element 220 is disposed against (e.g., in contact with) a sealing element 330 as discussed in further detail below. The engagement of the positioning elements 220 and the sealing element 330 prevents the device 200 from moving in a direction parallel to the longitudinal axis 101 of the plate 102. As axial movement between the device 200 and the packaging 302 is avoided, the device 200 and the packaging 302 do not rub against each other so as to create particles of the packaging material and/or the device material.

The lateral dimension D_{222L} of the positioning elements 220 and the diagonal dimension of the plate 102, when added, are greater than the lateral dimension (i.e., distance measured between two opposing corners 316 of the interior surface 308 of the sidewall 304) of the packaging 302.

When the device 200 is disposed in the packaging 302 and on the ledge 310, the device 200 and the packaging 302 are engaged by interference fit.

The device 200 and the packaging 302 collectively form a packaging assembly 300, as shown in FIG. 8, for transporting a plurality of medical containers.

When the device 200 lacking positioning elements according to the invention is disposed within the packaging 302, a space may be located between the interior surface 308 of the sidewall 304 and each edge 107 and corner 109 of the sidewall 108 of the plate 102. In such embodiments, there is insufficient contact between the sidewall 304 and the sidewall 108 and the device 200 can move in the packaging 302.

When the device 200 having the positioning elements 220 thereon is disposed within the packaging 302, the positioning element 220 may abut on the interior surface 308 of the sidewall 304. More particularly, an exterior surface 226 of each positioning element 220 may abut against the interior surface 308 of the sidewall 304.

A minority of a surface area of the exterior surface 226 is disposed against the interior surface 308 of the sidewall 304.

The exterior surface 226 may be curved as the positioning element 220 extends around the corner 109. A radius of curvature of the exterior surface 226 of the positioning elements 220 may be substantially equal to a radius of curvature of corners 316 of the sidewall 304 of the packaging 302. The curvature of the positioning elements 220 is visible in the top views of FIGS. 5A and 5B.

The engagement of the positioning elements 220 and the packaging 302 prevents the device 200 from moving in a direction perpendicular to the longitudinal axis 101 of the plate 102. As lateral movement between the device 200 and the packaging 302 is avoided, the device 200 and the packaging 302 do not rub against each other so as to create particles of the packaging material and/or the device material.

A sealing element 330 (FIGS. 3 and 7) may be coupled to the uppermost surface 306. The sealing element 330 is configured to close the opening 314 of the packaging 302 after the device 100, 200 is disposed therein and allow for sterilization of the packaging 302. The sealing element 330 is a microbial barrier. The sealing element 330 may be a porous microbial barrier. The sealing element 330 may also be permeable to a sterilization gas such that the interior of the packaging 302 can be sterilized after the sealing element 330 is disposed on the uppermost surface 306. The sterilization gas may be, for example, ethylene oxide. In an exemplary embodiment, the sealing element 330 may be formed of a TYVEK^{®} sheet.

In some embodiments, the second end 224 of the positioning element 220 has a rounded surface 228. The rounded surface 228 is configured to contact the sealing element 330 without piercing the sealing element 330 during transportation of the assembly 300.

In any of the foregoing embodiments, the positioning elements 120, 220 may be formed integrally with the plate 102. In such embodiments, the positioning elements 120, 220 and the plate 102 are formed together in a one-shot or two-shot injection molding process. Alternatively, the positioning elements 120 may be separately formed from the plate 102 and coupled thereto by adhesive or other coupling means. The positioning elements 120, 220 may be formed from the same or different polymeric material as the plate 102.

While the present disclosure has been described herein with respect to certain illustrated embodiments, those of ordinary skill in the art will recognize and appreciate that it is not so limited. The scope of the invention is defined by the appended claims.

## Claims

1. A device (100, 200) for supporting a plurality of medical containers, comprising:
a plate (102) having an upper surface (104), a lower surface (106), and a sidewall (108) defining a perimeter of the plate (102) and extending between the upper surface (104) and the lower surface (106), the sidewall (108) comprising four edges (107) intersecting at corners (109), a longitudinal axis (101) of the plate (102) extending perpendicular to the upper surface (104), the plate (102) defining a plurality of openings (110) extending through the plate (102) from the upper surface (104) to the lower surface (106), each opening (110) configured to retain a medical container therein, the plate (102) being dimensioned to be disposed in a packaging (302) for transporting a plurality of medical containers such that a distance measured between opposing edges (107) of the sidewall (108) is less than a distance measured between opposing sidewalls (304) of an interior surface (308) of the packaging (302);
wherein the device further comprises at least two positioning elements (120, 220) coupled at a first end (122, 222) thereof to the plate (102), each of the positioning elements (120, 220) having a second end (124, 224) opposite the first end (122, 222) detached from the plate (102), the first of the at least two positioning elements (120, 220) is at least partly disposed on a first edge of the four edges (107), the second of the at least two positioning elements (120, 220) is at least partly disposed on a second edge of the four edges (107), the first edge and second edge being adjacent to each other,
**characterized in that**
when the plate (102) is disposed in the packaging (302), each positioning element (120, 220) extends a lateral distance beyond the sidewall (108) of the plate (102) and axially beyond the upper surface (104) of the plate (102) such that the positioning element (120, 220) forms an acute angle relative to the longitudinal axis (101) of the plate (102) and such that each of the positioning elements (120, 220) contacts the interior surface (308) of the sidewall (304) of the packaging (302) when the plate (102) is disposed therein.

2. The device (200) of claim 1, wherein the first of the at least two positioning elements is disposed at a first corner (109), the second of the at least two positioning elements is disposed at a second corner (109), the first and second corners (109) being diagonally opposite each other.

3. The device (200) of claim 2, wherein each of the at least two positioning elements (220) is a rigid element configured to be displaced such that the acute angle (β) of the positioning element (102) is variable by about 1° when the plate (102) having the positioning elements (120) thereon is disposed within the packaging (302) and when the plate (102) is exterior to the packaging (302).

4. The device (200) of any one of claims 2-3, wherein the device comprises four positioning elements, and one positioning element (220) is coupled to each corner (109) of the sidewall (108).

5. The device (100) of claim 1, wherein the first of the at least two positioning elements is disposed exclusively on a first edge of the four edges (107), the second of the at least two positioning elements is disposed on a second edge of the four edges, the first edge and second edge being adjacent to each other, and the first positioning element being separate from the second position element.

6. The device (100) of claim 5, wherein the positioning element (120) is a flexible element configured to be displaced such that the acute angle (α) of the positioning element (102) is variable by 65° to 80° when the plate (102) having the positioning elements (120) thereon is disposed within the packaging (302) and when the plate (102) is exterior to the packaging (302).

7. The device (100) of any one of claims 5-6, wherein at least one positioning element (120) is coupled to each edge (107) of the sidewall (108).

8. The device (100, 200) of any one of claims 1-7, wherein the positioning elements (120, 220) are formed integrally with the plate (102).

9. A packaging system (300) for receiving a plurality of medical containers, comprising:
a packaging (302) comprising a sidewall (304) extending from a bottom wall (306), an interior surface (308) of the sidewall (304) being stepped so as to define a ledge (310);
the device (100, 200) of any of claims 1-8 comprising the plate (102) having the at least two positioning elements (120, 220) disposed in the packaging (302), wherein the lower surface (106) of the plate (102) is disposed on the ledge (310) and wherein the positioning elements (120, 220) are disposed on the interior surface (308) of the sidewall (304).

10. The packaging system (300) of claim 9, wherein a surface (226) of each of the at least two positioning elements (220) disposed on the interior surface (308) of the sidewall (304) has a curvature at corners (316) that corresponds to a curvature of the interior surface (308) of the plurality of sidewalls (304).

11. The packaging system (300) of claim 9 or 10, wherein a minority of a surface area of the surface (126, 226) of the positioning element (120, 220) is disposed against the interior surface (308) of the sidewall (304).

12. The packaging system (300) of any one of claims 9-11, wherein an axial dimension of each positioning element (220) is less than or equal to an axial dimension measured between the ledge (310) and an uppermost surface (312) of the packaging (302).

13. The packaging system (300) of any one of claims 9-12, wherein the plate (102) comprising the positioning elements (120, 220) engages the interior surface (308) of the sidewalls (304) of the packaging (302) by interference fit.

14. The packaging system (300) of any one of claims 9-13, wherein the device (100, 200) further comprises a plurality of chimneys (130), each chimney (130) extends circumferentially about an opening of the plurality of openings (110) and extends axially over the upper surface (104) of the plate (102), each chimney (130) configured to retain a medical container therein.

## Patentansprüche

1. Vorrichtung (100, 200) zum Stützen einer Vielzahl von medizinischen Behältern, umfassend:
eine Platte (102), die eine obere Oberfläche (104), eine untere Oberfläche (106) und eine Seitenwand (108) aufweist, die einen Umfang der Platte (102) definiert und sich zwischen der oberen Oberfläche (104) und der unteren Oberfläche (106) erstreckt, wobei die Seitenwand (108) vier Kanten (107) umfasst, die sich an Ecken (109) schneiden, eine Längsachse (101) der Platte (102) sich senkrecht zur oberen Oberfläche (104) erstreckt, die Platte (102) eine Vielzahl von Öffnungen (110) definiert, die sich durch die Platte (102) von der oberen Oberfläche (104) zur unteren Oberfläche (106) erstrecken, jede Öffnung (110) dazu ausgestaltet ist, einen medizinischen Behälter darin zurückzuhalten, die Platte (102) bemessen ist, um derart in einer Verpackung (302) zum Transportieren einer Vielzahl von medizinischen Behältern angeordnet zu werden, dass ein Abstand, der zwischen entgegengesetzten Kanten (107) der Seitenwand (108) gemessen wird, kleiner als ein Abstand ist, der zwischen entgegengesetzten Seitenwänden (304) einer inneren Oberfläche (308) der Verpackung (302) gemessen wird;
wobei die Vorrichtung ferner mindestens zwei Positionierungselemente (120, 220) umfasst, die an einem ersten Ende (122, 222) davon an die Platte (102) gekoppelt sind, wobei jedes der Positionierungselemente (120, 220) ein zweites Ende (124, 224) aufweist, das dem ersten Ende (122, 222) entgegengesetzt und von der Platte (102) gelöst ist, wobei das erste der mindestens zwei Positionierungselemente (120, 220) zumindest teilweise an einer ersten Kante von den vier Kanten (107) angeordnet ist, das zweite der mindestens zwei Positionierungselemente (120, 220) zumindest teilweise an einer zweiten Kante von den vier Kanten (107) positioniert ist und die erste Kante und die zweite Kante einander benachbart sind,
**dadurch gekennzeichnet, dass**, wenn die Platte (102) in der Verpackung (302) angeordnet ist, jedes Positionierungselement (120, 220) sich über eine seitliche Distanz über die Seitenwand (108) der Platte (102) hinaus und axial über die obere Oberfläche (104) der Platte (102) hinaus erstreckt, derart dass das Positionierungselement (120, 220) einen spitzen Winkel in Bezug auf die Längsachse (101) der Platte (102) bildet, und derart dass jedes der Positionierungselemente (120, 220) die innere Oberfläche (308) der Seitenwand (304) der Verpackung (302) berührt, wenn die Platte (102) darin angeordnet ist.

2. Vorrichtung (200) nach Anspruch 1, wobei das erste der mindestens zwei Positionierungselemente an einer ersten Ecke (109) angeordnet ist, das zweite der mindestens zwei Positionierungselemente an einer zweiten Ecke (109) angeordnet ist und die erste und die zweite Ecke (109) einander diagonal entgegengesetzt sind.

3. Vorrichtung (200) nach Anspruch 2, wobei jedes der mindestens zwei Positionierungselemente (220) ein starres Element ist, das dazu ausgestaltet ist, derart verlagert zu werden, dass der spitze Winkel (β) des Positionierungselements (102) um etwa 1° veränderlich ist, wenn die Platte (102), welche die Positionierungselemente (120) daran aufweist, innerhalb der Verpackung (302) angeordnet ist und wenn die Platte (102) sich außerhalb der Verpackung (302) befindet.

4. Vorrichtung (200) nach einem der Ansprüche 2 bis 3, wobei die Vorrichtung vier Positionierungselemente umfasst und ein Positionierungselement (220) an jede Ecke (109) der Seitenwand (108) gekoppelt ist.

5. Vorrichtung (100) nach Anspruch 1, wobei das erste der mindestens zwei Positionierungselemente ausschließlich an einer ersten Kante von den vier Kanten (107) angeordnet ist, das zweite der mindestens zwei Positionierungselemente an einer zweiten Kante von den vier Kanten angeordnet ist, die erste Kante und die zweite Kante einander benachbart sind und das erste Positionierungselement von dem zweiten Positionierungselement getrennt ist.

6. Vorrichtung (100) nach Anspruch 5, wobei das Positionierungselement (120) ein biegsames Element ist, das dazu ausgestaltet ist, derart verlagert zu werden, dass der spitze Winkel (α) des Positionierungselements (102) um 65° bis 80° veränderlich ist, wenn die Platte (102), welche die Positionierungselemente (120) daran aufweist, innerhalb der Verpackung (302) angeordnet ist und wenn die Platte (102) sich außerhalb der Verpackung (302) befindet.

7. Vorrichtung (100) nach einem der Ansprüche 5 bis 6, wobei mindestens ein Positionierungselement (120) an jede Kante (107) der Seitenwand (108) gekoppelt ist.

8. Vorrichtung (100, 200) nach einem der Ansprüche 1 bis 7, wobei die Positionierungselemente (120, 220) einteilig mit der Platte (102) gebildet sind.

9. Verpackungssystem (300) zum Aufnehmen einer Vielzahl von medizinischen Behältern, umfassend:
eine Verpackung (302), die eine Seitenwand (304) umfasst, die sich von einer unteren Wand (306) erstreckt, wobei eine innere Oberfläche (308) der Seitenwand (304) derart abgestuft ist, dass sie einen Absatz (310) definiert;
die Vorrichtung (100, 200) nach einem der Ansprüche 1 bis 8, welche die Platte (102) umfasst, welche die mindestens zwei Positionierungselemente (120, 220) aufweist, die in der Verpackung (302) angeordnet sind, wobei die untere Oberfläche (106) der Platte (102) auf dem Absatz (310) angeordnet ist und wobei die Positionierungselemente (120, 220) an der inneren Oberfläche (308) der Seitenwand (304) angeordnet sind.

10. Verpackungssystem (300) nach Anspruch 9, wobei eine Oberfläche (226) von jedem der mindestens zwei Positionierungselemente (220), die an der inneren Oberfläche (308) der Seitenwand (304) angeordnet sind, an Ecken (316) eine Krümmung aufweist, die einer Krümmung der inneren Oberfläche (308) der Vielzahl von Seitenwänden (304) entspricht.

11. Verpackungssystem (300) nach Anspruch 9 oder 10, wobei ein kleinerer Teil eines Flächeninhalts der Oberfläche (126, 226) des Positionierungselements (120, 220) gegen die innere Oberfläche (308) der Seitenwand (304) angeordnet ist.

12. Verpackungssystem nach einem der Ansprüche 9 bis 11, wobei eine axiale Abmessung jedes Positionierungselements (220) kleiner oder gleich einer axialen Abmessung ist, die zwischen dem Absatz (310) und einer obersten Oberfläche (312) der Verpackung (302) gemessen wird.

13. Verpackungssystem (300) nach einem der Ansprüche 9 bis 12, wobei die Platte (102), welche die Positionierungselemente (120, 220) umfasst, durch Presspassung mit der inneren Oberfläche (308) der Seitenwände (304) der Verpackung (302) ineinandergreift.

14. Verpackungssystem (300) nach einem der Ansprüche 9 bis 13, wobei die Vorrichtung (100, 200) ferner eine Vielzahl von Schächten (130) umfasst, wobei jeder Schacht (130) sich in Umfangsrichtung um eine Öffnung der Vielzahl von Öffnungen (110) erstreckt und sich axial über die obere Oberfläche (104) der Platte (102) erstreckt, wobei jeder Schacht (130) dazu ausgestaltet ist, einen medizinischen Behälter darin zurückzuhalten.

## Revendications

1. Dispositif (100, 200) destiné à supporter une pluralité de récipients médicaux, comprenant :
une plaque (102) ayant une surface supérieure (104), une surface inférieure (106) et une paroi latérale (108) définissant un périmètre de la plaque (102) et s'étendant entre la surface supérieure (104) et la surface inférieure (106), la paroi latérale (108) comprenant quatre bords (107) se coupant aux coins (109), un axe longitudinal (101) de la plaque (102) s'étendant perpendiculairement à la surface supérieure (104), la plaque (102) définissant une pluralité d'ouvertures (110) s'étendant à travers la plaque (102) depuis la surface supérieure (104) jusqu'à la surface inférieure (106), chaque ouverture (110) étant configurée pour retenir un conteneur médical à l'intérieur, la plaque (102) étant dimensionnée pour être disposée dans un emballage (302) destiné à transporter une pluralité de conteneurs médicaux de telle sorte qu'une distance mesurée entre des bords opposés (107) de la paroi latérale (108) soit inférieure à une distance mesurée entre des parois latérales opposées (304) d'une surface intérieure (308) de l'emballage (302) ;
le dispositif comprenant en outre au moins deux éléments de positionnement (120, 220) couplés à une première extrémité (122, 222) de ceux-ci à la plaque (102), chacun des éléments de positionnement (120, 220) ayant une deuxième extrémité (124, 224) opposée à la première extrémité (122, 222) détachée de la plaque (102), le premier des au moins deux éléments de positionnement (120, 220) est au moins partiellement disposé sur un premier bord des quatre bords (107), le second des au moins deux éléments de positionnement (120, 220) étant au moins partiellement disposé sur un second bord des quatre bords (107), le premier bord et le second bord étant adjacents l'un à l'autre,
**caractérisé en ce que** lorsque la plaque (102) est disposée dans l'emballage (302), chaque élément de positionnement (120, 220) s'étend sur une distance latérale au-delà de la paroi latérale (108) de la plaque (102) et axialement au-delà de la surface supérieure (104) de la plaque (102) de telle sorte que l'élément de positionnement (120, 220) forme un angle aigu par rapport à l'axe longitudinal (101) de la plaque (102) et de telle sorte que chacun des éléments de positionnement (120, 220) entre en contact avec la surface intérieure (308) de la paroi latérale (304) de l'emballage (302) lorsque la plaque (102) est disposée à l'intérieur.

2. Le dispositif (200) selon la revendication 1, dans lequel le premier des au moins deux éléments de positionnement est disposé au niveau d'un premier coin (109), le second des au moins deux éléments de positionnement est disposé au niveau d'un second coin (109), les premier et second coins (109) étant diagonalement opposés l'un à l'autre.

3. Le dispositif (200) selon la revendication 2, dans lequel chacun des au moins deux éléments de positionnement (220) est un élément rigide configuré pour être déplacé de telle sorte que l'angle aigu (β) de l'élément de positionnement (102) soit variable d'environ 1° lorsque la plaque (102) comportant les éléments de positionnement (120) est disposée à l'intérieur de l'emballage (302) et lorsque la plaque (102) est à l'extérieur de l'emballage (302).

4. Le dispositif (200) de l'une quelconque des revendications 2 à 3, dans lequel le dispositif comprend quatre éléments de positionnement, et un élément de positionnement (220) est couplé à chaque coin (109) de la paroi latérale (108).

5. Le dispositif (100) de la revendication 1, dans lequel le premier des au moins deux éléments de positionnement est disposé exclusivement sur un premier bord des quatre bords (107), le deuxième des au moins deux éléments de positionnement est disposé sur un deuxième bord des quatre bords, le premier bord et le deuxième bord étant adjacents l'un à l'autre, et le premier élément de positionnement étant séparé du deuxième élément de positionnement.

6. Le dispositif (100) selon la revendication 5, dans lequel l'élément de positionnement (120) est un élément flexible configuré pour être déplacé de telle sorte que l'angle aigu (α) de l'élément de positionnement (102) soit variable de 65° à 80° lorsque la plaque (102) comportant les éléments de positionnement (120) est disposée à l'intérieur de l'emballage (302) et lorsque la plaque (102) est à l'extérieur de l'emballage (302).

7. Le dispositif (100) de l'une quelconque des revendications 5 à 6, dans lequel au moins un élément de positionnement (120) est couplé à chaque bord (107) de la paroi latérale (108).

8. Le dispositif (100, 200) selon l'une quelconque des revendications 1 à 7, dans lequel les éléments de positionnement (120, 220) sont formés d'un seul tenant avec la plaque (102).

9. Système d'emballage (300) destiné à recevoir une pluralité de conteneurs médicaux, comprenant :
un emballage (302) comprenant une paroi latérale (304) s'étendant à partir d'une paroi inférieure (306), une surface intérieure (308) de la paroi latérale (304) étant étagée de manière à définir un rebord (310) ;
le dispositif (100, 200) de l'une quelconque des revendications 1 à 8 comprenant la plaque (102) ayant au moins deux éléments de positionnement (120, 220) disposés dans l'emballage (302), dans lequel la surface inférieure (106) de la plaque (102) est disposée sur le rebord (310) et dans lequel les éléments de positionnement (120, 220) sont disposés sur la surface intérieure (308) de la paroi latérale (304).

10. Système d'emballage (300) selon la revendication 9, dans lequel une surface (226) de chacun des au moins deux éléments de positionnement (220) disposés sur la surface intérieure (308) de la paroi latérale (304) présente une courbure au niveau des coins (316) qui correspond à une courbure de la surface intérieure (308) de la pluralité de parois latérales (304).

11. Le système d'emballage (300) selon la revendication 9 ou 10, dans lequel une minorité de la surface (126, 226) de l'élément de positionnement (120, 220) est disposée contre la surface intérieure (308) de la paroi latérale (304).

12. Le système d'emballage (300) de l'une quelconque des revendications 9 à 11, dans lequel une dimension axiale de chaque élément de positionnement (220) est inférieure ou égale à une dimension axiale mesurée entre le rebord (310) et une surface supérieure (312) de l'emballage (302).

13. Le système d'emballage (300) selon l'une quelconque des revendications 9 à 12, dans lequel la plaque (102) comprenant les éléments de positionnement (120, 220) s'engage avec la surface intérieure (308) des parois latérales (304) de l'emballage (302) par ajustement serré.

14. Le système d'emballage (300) selon l'une quelconque des revendications 9 à 13, dans lequel le dispositif (100, 200) comprend en outre une pluralité de cheminées (130), chaque cheminée (130) s'étendant circonférentiellement autour d'une ouverture de la pluralité d'ouvertures (110) et s'étendant axialement sur la surface supérieure (104) de la plaque (102), chaque cheminée (130) étant configurée pour retenir un récipient médical à l'intérieur.
